# EUROPEAN PATENT APPLICATION

(11) **EP 1 889 851 A1**
(43) Date of publication of application: **20.02.2008**
(21) Application number: 06017304.4
(22) Date of filing: 18.08.2006
(51) Int. Cl.: C07K 7/06, A61K 39/00

(54) **PAX2 and PAX8 as targets for immunologic and molecular tumour treatment strategies**

(71) Applicant: Charite Universitätsmedizin-Berlin, 10117 Berlin (DE)
(72) Inventor: Keilholz, Ulrich, Prof. Dr., 12203 Berlin (DE)
(74) Representative: Krauss, Jan

(57) **Abstract**

Briefly, the present invention refers to the transcription factors PAX2 and PAX8 expressed in solid tumours and haematologic malignancies, and their utility as a target in immunotherapy and molecular therapy. In more detail, the invention refers to a method for identifying an immunogenic T-cell epitope from PAX2 and/or PAX8. Furthermore, the invention refers to a use of immunogenic T-cell epitopes, e.g. identified by said method, and their use as targets for the recognition by targeting means, e.g. T-cells or antibodies. The invention also refers to peptides representing immunogenic T-cell epitopes and their uses for the preparation of a pharmaceutical composition for immunotherapy of PAX2 and/or PAX8 expressing malignancies.

## Description

Briefly, the present invention refers to the transcription factors PAX2 and PAX8 expressed in solid tumours and haematologic malignancies, and their utility as a target in immunotherapy and molecular therapy. In more detail, the invention refers to a method for identifying an immunogenic T-cell epitope from PAX2 and/or PAX8. Furthermore, the invention refers to a use of immunogenic T-cell epitopes, e.g. identified by said method, and their use as targets for the recognition by targeting means, e.g. T-cells or antibodies. The invention also refers to peptides representing immunogenic T-cell epitopes and their uses for the preparation of a pharmaceutical composition for immunotherapy of PAX2 and/or PAX8 expressing malignancies.

### Background of the invention

The transcription factor WT1 is an antigen, which has recently attracted much interest as target for cancer immunotherapy (1). Originally, WT1 was connected with Wilms' tumour, an embryonal malignancy of the kidney affecting infants and young children. Today it is established that WT1 is highly expressed in leukaemic blasts and is also broadly expressed in solid tumours. Recent papers show its wide expression in various solid tumour samples, including common malignancies as colorectal cancer, breast cancer and lung cancer. WT1 plays a key pathogenic role as down-regulation of the wt1 gene leads to growth arrest, differentiation and apoptosis. In patients with leukaemia and solid tumours frequently spontaneous T-cell responses to WT1 occur (2). We and others have started first clinical vaccination trials with WT1 peptides in patients with acute leukaemia showing good immunogenicity and clinical efficacy of the vaccine (3,4,5).

The reason for the overexpression of wt1 gene has been largely unclear. Mutations of WT1 have not or only rarely been found. The overexpressed protein is functionally active, which supports oncogenic function of WT1 but also traces probable malfunction to the elements lying upstream of wt1, which could include mutation of promoters or aberrant expression of its regulators.

There are three main physiological regulators of WT1 expression: PAX2, PAX8 and GATA1 proteins. PAX2 and PAX8, which are closely related transcription factors, are expressed in WT and are potentially involved in its induction. The PAX (paired axial) genes belong to the hombeobox gene family of transcription factors. These proteins have sequence homology to the *Drosophila* segmentation genes *paired* and *gooseberry.* The mammalian PAX proteins are numbered 1-9, all containing the so-called "paired box" sequence, a 124 amino-acid conserved domain. PAX2 and PAX proteins are closely related Both PAX2 and PAX8 genes play a role in the regulation of WT1 expression in the developing kidney. Induced overexpression of both PAX genes results in subsequent WT1 up-regulation (6,7). On the other hand there seems to be a feedback loop in which up-regulated WT1 may repress PAX2 expression. PAX genes are frequently expressed in cancer and often required for cancer survival (8). PAX2 and PAX8 are also expressed in various types of cancer including lymphomas, which do not express WT1. We could recently show that expression of the transcription factors PAX2 or PAX8 is a cause of up-regulation of WT1 in human acute myeloid leukaemia (AML), breast and colorectal carcinomas (9,10). Therefore, in addition to WT1 as target for immunotherapy itself, it is of great interest to exploit the WT1 expression regulators PAX2 and PAX8 as cancer vaccination antigen.

The patent US 6,723,506 discloses the finding of a fusion oncogene designated PAX8-PPARγ1 in carcinoma samples. The fusion oncogene (or its reciprocal PPARγ1-PAX8) is the result of a chromosomal translocation fusing chromosomes 2 and 2. Molecular characterization of PAX8-PPARγl revealed nucleotide and amino acid sequences useful for detection and treatment of certain tumours, particularly thyroid follicular carcinomas.

The patent US 6,071,697 provides a method for testing the differentiation status of pancreatic cells in mammals using two members of the PAX family, namely PAX4 and PAX6. A deficiency in PAX4 expression is indicative of deficiency or failure in β-cell development and thus insulin production. Accordingly, the method is useful for determining the risk of developing juvenile diabetes. Further disclosed are a method for testing a medicament for a gene therapy approach and transgenic mammals comprising an inactivated PAX4 allele and optionally and inactivated PAX6 allele.

The patent US 6,514,712 provides a monoclonal or polyclonal antibody which specifically binds the PAX9 antigen. It is also mentioned that members of the family of PAX genes, namely PAX1, PAX2, PAX3, PAX6, and PAX8, have been identified as protooncogenes due to their tumourigenic properties.

Cancer immunotherapeutic strategies are based on the generation or enhancement of an immune response to tumour-associated antigens (TAAs). A prerequisite for successful immunotherapy is the knowledge of immunogenic T-cell epitopes. The search for new T-cell epitopes in known TAAs using the classical "reverse immunology" strategy has led to the identification of several T-cell epitopes, most of them restricted to HLA-A2 (also referred to as HLA-A*02) (11). This strategy includes the prediction of potential T-cell epitopes from known tumour antigens, their analysis for MHC-binding, followed by the *in vitro* generation of peptide-reactive T-cells and their testing of target cell recognition. This strategy is rather laborious because of the need for T-cell induction against multiple peptides, which are often not processed. However, recognition of TAAs through T-cells requires processing in an antigen presenting cell und presentation together with a HLA molecule. In the setting of non-HLA-A2 (non-HLA-A*0201) alleles target tumour cells expressing both the respective HLA allele and the target antigen are often not readily available.

A more recent approach to increase the efficacy of identifying potential T-cell epitopes is the *ex vivo* screening of candidate epitopes for recognition by T-cells from patients which are naturally primed against the target antigen (12-15). Candidate epitopes can be predicted using appropriate prediction models *in silico* (e.g. ref. 16). WO 00/18795 used the BIMAS HLA peptide binding prediction analysis (17). An elegant approach to show epitope processing is by *in vitro* proteasome-mediated digestion pattern analysis. This approach has reliably identified HLA-A2 binding epitopes from tumour and viral proteins using 25-30-mer peptides encompassing the putative epitope (18,19). Even though such *in silico* prediction models as mentioned above may be highly efficacious to predict candidate epitopes, the necessity remains to verify their utility *in vitro* and *in vivo.*

An object of the present invention is to provide immunogenic T-cell epitopes useful in immunotherapy of malignancies.

### Summary of the invention

The object of the present invention is solved by an immunogenic T-cell epitope represented by a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

In one embodiment, the immunogenic T-cell epitope is from PAX2 and/or PAX8, preferably from PAX2.

The object of the present invention is further solved by a use of an immunogenic T-cell epitope from PAX2 and/or PAX8, preferably from PAX2, as a target for recognition by a targeting means.

In one embodiment, the T-cell epitope is a HLA binding T-cell epitope, preferably selected from a HLA-A2, HLA-A1 or HLA-A24 binding T-cell epitope.

In one embodiment, the T-cell epitope is represented by a peptide selected from the group of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

In one embodiment, the T-cell epitope is represented by the peptide according to SEQ ID No. 5 (3496).

In an alternative embodiment, three T-cell epitopes are used represented by the peptides according to SEQ ID No. 16 (3520), SEQ ID. No 29 (3550), and SEQ ID No. 32 (3553).

In another alternative embodiment, two T-cell epitopes are used represented by the peptides according to SEQ ID No. 5 (3496) and SEQ ID No. 6 (3497).

In just another alternative embodiment, two T-cell epitopes are used represented by the peptides according to SEQ ID No. 20 (3519) and SEQ ID No. 21 (3518).

In one embodiment, the targeting means is a component of the immune system, preferably a T-cell or an antibody.

In a preferred embodiment, the antibody is selected from the group consisting of monoclonal antibodies, polyclonal antibodies, homobodies, epibodies, Fc fragments and portions or variants thereof.

The object of the present invention is further solved by a use of a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553) for the preparation of a pharmaceutical composition for the treatment of a PAX2 and/or PAX8 expressing disease, preferably a PAX2 expressing disease.

In one embodiment, the disease is selected from renal cancer, colorectal cancer, breast cancer, and ovarian cancer.

In one embodiment, the peptide according to SEQ ID No. 5 (3496) is used for the preparation, and the disease preferably is renal cancer.

In an alternative embodiment, the peptides according to SEQ ID No. 16 (3520), SEQ ID No. 29 (3550), and SEQ ID No. 32 (3553) are used for the preparation, and the disease preferably is colorectal cancer.

In another alternative embodiment, the peptides according to SEQ ID No. 5 (3496) and 3497 SEQ ID No. 6 (3497) are used for the preparation, and the disease preferably is colorectal cancer.

In just another alternative embodiment, the peptides according to SEQ ID No. 20 (3519) and SEQ ID No. 21 (3518) are used for the preparation, and the disease preferably is colorectal cancer.

The object of the present invention is further solved by a use of a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553) for the preparation of a pharmaceutical composition for raising an immune response in a subject, preferably a mammal.

In one embodiment, the peptide according to SEQ ID No. 5 (3496) is used for the preparation.

In an alternative embodiment, the peptides according to SEQ ID No. 16 (3520), SEQ ID No. 29 (3550), and SEQ ID No. 32 (3553) are used for the preparation.

In another alternative embodiment, the peptides according to SEQ ID No. 5 (3496) and 3497 SEQ ID No. 6 (3497) are used for the preparation.

In just another alternative embodiment, the peptides according to SEQ ID No. 20 (3519) and SEQ ID No. 21 (3518) are used for the preparation.

In one embodiment, the immune response is raised by contacting the pharmaceutical composition with T-cells of a subject, preferably a mammal*, in vivo.*

In an alternative embodiment, the immune response is raised by contacting the pharmaceutical composition with T-cells of a subject, preferably a mammal*, ex vivo.*

The object of the present invention is further solved by a pharmaceutical composition comprising a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

In one embodiment, the pharmaceutical composition comprises a peptide according to SEQ ID No. 5 (3496).

In an alternative embodiment, the pharmaceutical composition comprises three peptides according to SEQ ID No. 16 (3520), SEQ ID No. 29 (3550), and SEQ ID No. 32 (3553).

In another alternative embodiment, the pharmaceutical composition comprises two peptides according to SEQ ID No. 5 (3496) and 3497 SEQ ID No. 6 (3497).

In just another alternative embodiment, the pharmaceutical composition comprises two peptides according to SEQ ID No. 20 (3519) and SEQ ID No. 21 (3518).

The object of the present invention is further solved by a use of a targeting means according to the present invention for the preparation of a pharmaceutical composition for the treatment of a PAX2 and/or PAX8-expressing disease, preferably a PAX2-expressing disease, most preferably selected from renal cancer, colorectal cancer, breast cancer, and ovarian cancer.

In one embodiment, the targeting means is a T-cell or an antibody directed against an immunogenic T-cell epitope from PAX2 and/or PAX8, preferably from PAX2.

In one embodiment, the T-cell epitope is represented by a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

In a preferred embodiment, the antibody is selected from the group consisting of monoclonal antibodies, polyclonal antibodies, homobodies, epibodies, Fc fragments and portions or variants thereof.

The object of the present invention is further solved by a pharmaceutical composition comprising a targeting means according to the present invention.

In one embodiment, the targeting means is a T-cell or an antibody directed against an immunogenic T-cell epitope from PAX2 and/or PAX8, preferably from PAX2.

In one embodiment, the T-cell epitope is represented by a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

In one embodiment, the targeting means is a T-cell or an antibody, directed against a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

In a preferred embodiment, the antibody is selected from the group consisting of monoclonal antibodies, polyclonal antibodies, homobodies, epibodies, Fc fragments and portions or variants thereof.

The object of the present invention is further solved by a method for identifying an immunogenic T-cell epitope, a portion or a variant thereof comprising the following steps:
(a) predicting a candidate epitope from PAX2 and/or PAX8, preferably from PAX2;
(b) *ex vivo* screening of one or more candidate epitopes predicted in step (a) for recognition by T-cells; and
(c) *in vitro* generating of T-cell clones.

In one embodiment, the T-cell epitope is a HLA binding T-cell epitope, preferably selected from HLA-A2, HLA-A1 and HLA-A24 binding T-cell epitope.

In one embodiment, the candidate epitope is excluded from the highly conserved paired box sequence of PAX2 and/or PAX8, preferably from PAX2.

In one embodiment, the T-cells in step (b) are obtained from a tumour patient having a PAX2 and/or PAX8-expressing disease, preferably a PAX2-expressing disease, most preferably selected from renal cancer, colorectal cancer, breast cancer, and ovarian cancer .

In one embodiment, the T-cell clones are useful for demonstrating natural epitope processing.

The object of the present invention is further solved by a use of the method according to the present invention for identifying an immunogenic T-cell epitope from PAX2 and/or PAX8, preferably from PAX2.

The object of the present invention is further solved by a use of the immunogenic T-cell epitope from PAX2 and/or PAX8, preferably from PAX2, identified by the method according to the present invention, as a target for a targeting means.

The object of the present invention is further solved by a use of an immunogenic T-cell epitope from PAX2 and/or PAX8, preferably from PAX2, identified by the method according to the present invention, for the preparation of a pharmaceutical composition for the treatment of a PAX2 and/or PAX8-expressing disease, preferably a PAX2-expressing disease.

The object of the present invention is further solved by a pharmaceutical composition comprising an immunogenic T-cell epitope from PAX2 and/or PAX8, preferably from PAX2, identified by the method according to the present invention.

The wording "use of an immunogenic T-cell epitope (...) as a target for recognition by a targeting means" may have a dual meaning. First, the T-cell epitope, e.g. represented by a peptide, is administered in order to raise an immune response in a subject, i.e. the exogenous T-cell epitope serves a an antigenic target for endogenous components of the immune system. Second, the T-cell epitope is part of endogenous PAX2 and/or PAX 8, and as such serves as a target for endogenous components of the immune system, e.g. T-cells and antibodies, which were raised previously in a subject in response to a contact with the administered T-cell epitope.

The wording "use (...) of a pharmaceutical composition for raising an immune response" comprises the meaning that the pharmaceutical composition is used as a vaccine for active immunisation. Alternatively, a pharmaceutical composition comprising a targeting means, e.g. T-cells or an antibody generated *in vitro,* can be used as for passive immunisation.

The wording "contacting the pharmaceutical composition with T-cells (...) *in vivo"* means that the pharmaceutical composition is administered to a subject, e.g. by sequential s.c. injections.

The wording "contacting the pharmaceutical composition with T-cells (...) *ex vivo"* comprises the meaning that T-cells are removed from a subject, contacted with the pharmaceutical composition *in vitro,* and finally infused into a subject (so-called T-cell therapy). Donor and acceptor of the T-cells may be identical or may be different. Furthermore, the donor and acceptor species may be identical or may be different.

In summary, the present invention refers to two transcription factors, PAX2 and PAX8 (paired box genes 2 and 8), expressed in various cancers and haematologic malignancies and their utility as a target in immunotherapy and molecular therapy. In more detail, the invention refers to the PAX proteins excluding the highly conserved paired box sequence (PAX2 AA 16-141, PAX 8 9-216) comprising T-cell epitopes or a portion or variant thereof.

Based on the knowledge that expression of the transcription factors PAX2 or PAX8 is a cause of up-regulation of WT1 in human AML, breast and colorectal carcinomas (9,10), we set out to provide tools and their uses for cancer immunotherapy and other therapies targeting PAX2 and PAX8.

In this study, we have identified HLA-A2 binding candidate epitopes which are spontaneously immunogenic in colorectal cancer patients. For this purpose, we have evaluated a strategy to directly identify immunogenic HLA-A2 binding T-cell epitopes from PAX2 combining the following steps: 1) Prediction of potential epitopes by the SYFPEITHI algorithm, 2) screening of potential epitopes for recognition by T-cells from tumour patients with PAX2-expressing malignancies, and 3) *in vitro* generation of T-cell clones as tools to demonstrate natural epitope processing by tumour cell lines. We first focused on HLA-A2 because of its relatively high prevalence (45%) among the Caucasian population and the high predictive value of the prediction algorithm used for this allele (16).

The unique sequence of the 416 amino acid PAX2 protein is located at amino acids 145 to 191. The highly conserved portion of the paired box domain (amino acids 16 to 140) and the homeobox (amino acids 235 to 278) are excluded from epitope identification. In the 450 amino acid PAX8 protein, the paired box is located at amino acids 9 to 216, amino acids 228 to 250 form the homeobox homology region, and a further highly conserved octapeptide sequence that is excluded from epitope identification is located at amino acids 180 to 187.

An approach to efficiently identify potential T-cell epitopes is the *ex vivo* screening of candidate epitopes for recognition by T-cells from patients which are naturally primed against the target antigen (20-22). Using this approach we could identify HLA-A2 binding candidate epitopes from PAX2 against which patients with colorectal cancer have spontaneous T cell responses.

Furthermore, using the world-wide-web databases for epitope prediction SYFPEITHI (www.syfpeithi.de) and BIMAS (http://bimas.cit.nih.gov/molbio/hla_bind/) and the database PAProC for prediction of proteasomal cleavage sites (www.paproc.de) we have predicted potential T-cell epitopes from PAX2 and PAX8 for various common HLA class I and II antigens, i.e. HLA-A1/A3/A24B7/b8/B44, DRB1*0101/+0401/+0701-binding candidate epitopes.

### Detailed description of the invention

### EXAMPLE 1: Identification of HLA-A2 positive PAX2 peptides as targets for T-cell recognition in cancer patients

### Patients and healthy controls

Peripheral blood mononuclear cells (PBMC) from 19 HLA-A2 positive colorectal cancer patients and 10 healthy subjects were collected and cryopreserved. The investigation had been approved by the Institutional Ethics Committee and informed consent was obtained from all individuals.

### Prediction of HLA-A2 binding peptides

Prediction of candidate 9-mer and 10-mer epitopes according to the HLA-A2 motif from PAX2 (swissprot accession no. AAC63385) was performed using the SYFPEITHI algorithm, and 11-to 13-mer candidate epitopes, 37 epitopes in total (SEQ ID No. 1 to 37) were calculated according to the same prediction model as described (16). Using this approach, 13 peptides (SEQ ID No. 1 to 6, 16, 20 to 22, 26, 29, and 32) were selected as candidate T-cell epitopes (Table I, see left column).

**Table I: HLA-A2 (HLA-A*0201) binding candidate epitopes (nona- and decamers) from PAX2 predicted using the SYFPEITHI algorithm**

| **Peptide No. SEQ ID No.** | **Position** | **Amino acid sequence** | **PAProC prediction of proteasomal digestion** | | | **Score** |
|---|---|---|---|---|---|---|
| | | | **WT Prot** | **Immuno** | **Const** | |
| 3469 SEQ ID No. 1 | 295§ | A L T P G L D E V | I | - | -/+ | 31 |
| 3480 SEQ ID No. 2 | 292§ | S L P A L T P G L | - | +/- | +/- | 27 |
| 3485 SEQ ID No. 3 | 299§ | G L D E V K S S L | I;II | - | +/- | 25 |
| 3490 SEQ ID No. 4 | 222 | H L V W T L R D V | - | +/- | -/+ | 24 |
| 3496 SEQ ID No. 5 | 337§ | T L P G Y P P H V | 1 | + | -/+ | 24 |
| 3497 SEQ ID No. 6 | 158* | V T A P G H T I V | - | + | +/- | 21 |
| SEQ ID No.7 | 185* | Y S I N G I L G I | I | - | +/- | 20 |
| SEQ ID No.8 | 219§ | G G L H L V W T L | II; III | -/+ | - | 20 |
| SEQ ID No.9 | 357 | T L A G M V P G S | - | - | - | 20 |
| SEQ ID No. 10 | 216 | R G G G G L H L V | - | +/- | + | 19 |
| SEQ ID No. 11 | 169* | T A S P P V S S A | I;II;III | +/- | -/+ | 17 |
| SEQ ID No. 12 | 186* | S I N G I L G I P | - | +/- | +/- | 17 |
| SEQ ID No. 13 | 213 | A H I R G G G G L | - | -/+ | +/- | 17 |
| SEQ ID No. 14 | 215 | I R G G G G L H L | - | - | - | 17 |
| SEQ ID No. 15 | 307 | L S A S T N P E L | - | + | - | 17 |
| 3520 SEQ ID No. 16 | 157* | G V T A P G H T I V | II | -/+ | +/- | 16 |
| SEQ ID No. 17 | 164* | T I V P S T A S P | I | - | +/- | 16 |
| SEQ ID No. 18 | 314 | E L G S N V S G T | I | +/- | - | 16 |
| SEQ ID No. 19 | 399 | S A A P R S A P A | - | - | +/- | 16 |
| 3519 SEQ ID No. 20 | 306 | S L S A S T N P E L | - | + | - | 25 |
| 3518 SEQ ID No. 21 | 226 | T L R D V S E G S V | - | +/- | - | 23 |
| 3516 SEQ ID No. 22 | 214 | H I R G G G L H L | - | -/+ | - | 21 |
| SEQID No.23 | 215 | I R G G G L H L V | III | - | -/+ | 19 |
| SEQ ID No. 24 | 287 | Q G N E Y S L P A L | III | +/- | -/+ | 19 |
| SEQ ID No. 25 | 294 | P A L T P G L D E V | III | - | -/+ | 19 |
| SEQ ID No. 26 | 310 | S T N P E L G S N V | III | -/+ | -/+ | 19 |
| SEQ ID No. 27 | 336 | T T L P G Y P P H V | - | -/+ | -/+ | 19 |
| SEQ ID No. 28 | 391 | L L S S P Y Y Y S A | - | - | -/+ | 19 |
| 3550 SEQ ID No. 29 | 168* | S T A S P P V S S A | I | - | - | 18 |
| SEQ ID No. 30 | 221 | L H L V W T L R D A | - | - | -/+ | 18 |
| SEQ ID No. 31 | 291 | Y S L P A L T P G L | - | - | - | 18 |
| 3553 SEQ ID No. 32 | 165* | I V P S T A S P P V | - | + | +/- | 17 |
| SEQIDNo.33 | 218 | G G G L H L V W T L | II | -/+ | - | 17 |
| SEQIDNo.34 | 318 | N V S G T Q T Y P V | - | - | -/+ | 17 |
| SEQ ID No. 35 | 184* | S Y S I N G I L G I | I | - | - | 16 |
| SEQIDNo.36 | 357 | T L A G M V P G S E | - | - | -/+ | 16 |
| SEQ ID No. 37 | 399 | S A A P R S A P A A | II | - | +/- | 16 |

| | | | | | | |
|---|---|---|---|---|---|---|
| * within unique sequence of PAX2 § high BIMAS-score (other algorithm) | | | | | | |

### Peptide synthesis

Peptides were synthesized in an Applied Biosystems 432A peptide synthesizer following standard protocols. Synthesized products were analysed by high-performance liquid chromatography (Varian Star; Zinsser Analytics, Munich, Germany) and MALDI-TOF mass spectometry (G2025A; Hewlett-Packard, Waldbronn, Germany), and purified by preparative HPLC to purities >95%.

### T-cell analysis

Antigen-specific T-cells were detected by a functional assay. Intracellular IFNy accumulation induced by WT1 peptide was assessed by flow cytometry as described previously (2).

### Screening of T-cell reactivity to predicted epitopes in colorectal cancer patients

Using functional flow cytometry detecting peptide-induced induction of intracellular IFNy we analyzed circulating T-cells from 19 HLA-A2 positive colorectal cancer patients for recognition of the 13 PAX2 candidate epitopes.

Results of the flow cytometric analysis of T-cell responses to HLA-A2 binding candidate epitopes from PAX2 in the 19 patients are summarized in Table II. Frequencies of specific CD3⁺CD8⁺ T-cells secreting IFNγ in response to a pool of 2 or 3 PAX2 peptides as well as to an irrelevant HLA-A2 binding peptide from HIV are shown.

Of 19 HLA-A2 positive patients analysed, 5 patients showed a T-cell response against a mixture of the three peptides 3520 (SEQ ID No. 16), 3550 (SEQ ID. No. 29), and 3553 (SEQ ID No. 32) (Table II, see right column). Two further patients showed a T-cell response against a mixture of two peptides, namely peptides 3496 (SEQ ID No. 5) and 3497 (SEQ ID No. 6), and peptides 3519 (SEQ ID No. 20) and 3518 (SEQ ID No. 21), respectively.

No T-cell response against these peptides was observed in 10 healthy HLA-A2 positive subjects (Table III). These findings suggest that similar to WT1, PAX2 may be recognized in tumour-bearing patients.

**Table II: T-cell response to HLA-A2 (HLA-A*0201) binding PAX2 candidate epitopes in 19 patients with colorectal cancer (IFN-γ plus T-cells in % of CD3⁺CD8⁺ T-cells detected by intracellular staining)**

| **Peptide No.** | **Hiv** | **3469+80** | **3485+90** | **3496+97** | **3516+48** | **3518+19** | **3550+3+20** |
|---|---|---|---|---|---|---|---|
| # 1 | 0.15 | 0.06 | 0.13 | 0.17 | 0.16 | 0.17 | **0.35** |
| # 2 | 0.37 | 0.29 | 0.18 | 0.32 | 0.63 | 0.34 | 0.33 |
| # 3 | 0.25 | 0.19 | 0.19 | 0.15 | 0.25 | 0.47 | 0.27 |
| # 4 | 0.08 | 0.03 | 0.04 | 0.05 | 0.08 | 0.04 | **0.16** |
| # 5 | 0.03 | 0.03 | 0.03 | 0.05 | 0.04 | 0.01 | **0.1** |
| # 6 | 0.09 | 0.1 | 0.09 | 0.09 | 0.11 | 0.05 | 0.05 |
| # 7 | 0.06 | 0.04 | 0.03 | 0.04 | 0.05 | 0.04 | **0.12** |
| # 8 | 0.32 | 0.1 | 0.06 | 0.07 | 0.08 | 0.11 | 0.08 |
| # 9 | 0.2 | 0.11 | 0.18 | 0.03 | 0.16 | 0.06 | 0.05 |
| # 10 | 0.2 | 0.2 | 0.1 | 0.25 | 0.16 | 0.22 | 0.2 |
| # 11 | 0.24 | 0.13 | 0.11 | 0.11 | 0.25 | 0.17 | 0.2 |
| # 12 | 0.24 | 0.11 | 0.04 | 0.17 | 0.2 | 0.1 | 0.12 |
| # 13 | 0.13 | 0.08 | 0.13 | 0.07 | 0.1 | 0.16 | 0.08 |
| # 14 | 0.61 | 0.18 | 0.19 | 0.43 | 0.42 | 0.87 | 0.7 |
| # 15 | 0.1 | 0.11 | 0.14 | 0.15 | 0.04 | **0.25** | 0.19 |
| # 16 | 0.09 | 0.1 | 0.08 | **0.26** | 0.13 | 0.12 | 0.11 |
| # 17 | 0.25 | 0.03 | 0.44 | 0.09 | 0.35 | 0.05 | 0.09 |
| # 18 | 0.23 | 0.17 | 0.16 | 0.06 | 0.17 | 0.35 | 0.12 |
| # 19 | 0.08 | 0.03 | 0.08 | 0.07 | 0.03 | 0.06 | **0.21** |

**Table III: T-cell response to HLA-A2 (HLA-A*0201) binding PAX2 candidate epitopes in 10 healthy donors (IFN-γ plus T-cells in % of CD3⁺CD8⁺ T-cells)**

| **Donor No.** | **Hiv A2** | **3469 + 3480** | **3485+ 3490** | **3496+ 3497** | **3516+ 3548** | **3518+ 3519** | **3550+ 03+20** | **All PAX peptides** | **PMA/ IONO** |
|---|---|---|---|---|---|---|---|---|---|
| # 1 | 0.02 | 0.02 | 0.00 | 0.02 | 0.02 | 0.06 | 0.04 | | 41.43 |
| # 2 | 0.07 | 0.01 | 0.03 | 0.01 | 0.01 | 0.02 | 0.03 | | 24.77 |
| # 3 | 0.01 | 0.01 | 0.03 | 0.03 | 0.02 | 0.00 | 0.03 | | 14.87 |
| # 4 | 0.02 | 0.01 | 0.09 | 0.01 | 0.00 | 0.03 | 0.01 | 0.03 | 75.73 |
| # 5 | 0.01 | 0.01 | 0.01 | 0.00 | 0.00 | 0.00 | 0.02 | 0.02 | 19.37 |
| # 6 | 0.01 | 0.01 | 0.01 | 0.01 | 0.00 | 0.00 | 0.00 | | 77.87 |
| # 7 | 0.01 | | | | | | | 0.00 | 24.53 |
| # 8 | 0.02 | | | | | | | 0.01 | 22.25 |
| # 9 | 0.02 | 0.01 | 0.05 | 0.02 | 0.02 | 0.02 | 0.03 | 0.00 | 18.02 |
| # 10 | 0 | | | | | | | 0.00 | 6.59 |

These data show for the first time that peptides derived from PAX2 epitopes, and thus probably also peptides derived from PAX8 epitopes, have the potential to induce a T-cell response. Thus, immunogenic T-cell epitopes of PAX2, and probably of PAX8, can serve as targets in immunotherapy.

### EXAMPLE 2: Induction of a T-cell response in a cancer patient

In a patient with renal cancer we were able to induce specific T-cells against the peptide 3496 (SEQ ID No. 5), and could detect by Cr release assay specific cytolysis of both T2-cells loaded with 3496 peptide (32% killing at 10:1 E:T versus 8% killing of HIV peptide loaded T2) and colon cancer cell line SW480 (28% which could be inhibited by cold target to 10%). A panel of colorectal cancer cell lines has been characterized expressing both PAX2 and PAX8 to function as targets for recognition by specific T-cells (Table IV).

**Table IV: PAX2 and PAX8 expression in colorectal cancer cell lines (ratio PAX/PBGD determined by quantitative RT-PCR)**

| **Cell line** | **Expression PAX2** | **Expression PAX8** |
|---|---|---|
| Colo 205 | 1.64E-04 | 2.00E-04 |
| Colo 206F | 3.98E-04 | 3.76E-05 |
| Colo 320 | 1.60E-05 | 3.29E-05 |
| Cx 2 | 1.68E-04 | 1.88E-05 |
| Cx 94 | 3.93E-03 | 2.22E-05 |
| DLD 1 | 1.00E-08 | 3.18E-05 |
| HCT 116 | 2.62E-02 | 7.00E-05 |
| HT 29 | 1.13E-04 | 3.89E-05 |
| SW 403 | 8.96E-06 | 1.64E-04 |
| SW 480 | 1.24E-04 | 7.19E-05 |
| SW 620 | 2.69E-04 | 4.01E-05 |
| SW 948 | 1.10E-04 | 2.13E-05 |
| CaCo 2 | 2.24E-05 | 2.49E-06 |
| LST 174 | 1.00E-08 | 1.28E-05 |

These data confirm that peptides derived from PAX2 epitopes, and thus probably also peptides derived from PAX8 epitopes, are indeed able to raise a T-cell response in a subject.

### EXAMPLE 3: PAX2 vaccination protocol

For vaccination with PAX2 or PAX8 epitopes, the protocol given in (1) describing the vaccination with WT1.126-134 in a HLA-A2 (HLA-A*0201) positive patient is applied.

An HLA-A2 positive patient diagnosed with PAX2 positive cancer receives eight biweekly vaccinations with the peptide in a dose of 0.2 mg admixed with 1 mg keyhole limpet hemocyanin (KLH, Immucothel, biosyn, Germany) as adjuvant i.d. and s.c. on day 0. GM-CSF (Leukomax, Essex Pharma, Germany) in a dose of 75 µg per day is injected s.c. at the same site (proximal thigh) as the WT peptide on days -2 to +1. The combination of both ajuvants is chosen due to the immunological efficacy in melanoma peptide vaccination (23). Vaccination cycles can be repeated if necessary.

During the course of therapy, the patient is monitored with respect to common diagnostic parameters such as blood counts and clinical biochemistry. T-cell response to PAX2 peptides is performed in a peripheral blood using tetramer and intracellular IFNγ staining by flow cytometry (2).

### References

1. Keilholz U, Menssen HD, Gaiger A et al. Wilms' tumour gene 1 (WT1) in human neoplasia. Leukemia 19:1318-23; 2005.
2. Scheibenbogen C, Letsch A, Thiel E et αl. CD8 T-cell responses to Wilms tumor gene product WT1 and proteinase 3 in patients with acute myeloid leukemia. Blood 100:2132-2137; 2002.
3. Mailander V, Scheibenbogen C, Thiel E et al. Complete remission in a patient with recurrent acute myeloid leukemia induced by vaccination with WT1 peptide in the absence of hematological or renal toxicity. Leukemia 18:165-166; 2004.
4. Oka Y, Tsuboi A, Taguchi T et al. Induction of WT1 (Wilms' tumor gene)-specific cytotoxic T lymphocytes by WT1 peptide vaccine ad the resultant cancer regression. Proc Natl Acad Sci USA 101:13885-13890; 2004.
5. Rosenfeld C, Cheever MA and GaigerA. WT1 in acute leukaemia, chronic myelogenous and myelodysplastic syndrome: therapeutic potential of WT1 targeted therapies. Leukemia 17:1301-1312; 2003.
6. Dehbi M and Pelletier J. PAX8-mediated activation of the wt1 tumor suppressor gene. EMBO J 15:4297-4306; 1996.
7. Dehbi M, Ghahremani M, Lechner M et al. The paired-box transcription factor, PAX2, positively modulates expression of the Wilms' tumor suppressor gene (WT1). Oncogene 13:447-453; 1996.
8. Muratovska A, Zhou C and He S. Paired-box genes are frequently expressed in cancer and often required for cancer survival. Oncogene 22:7989-7997; 2003.
9. Siehl J, Thiel E, Heufelder K et al. Possible regulation of Wilm's tumor gene 1 (WT1) expression by the paired box genes PAX2 and PAX8 and by the haematopoietic transcription factor GATA-1 in human acute myeloid leukemias. Br J Hematol 123:235-242; 2003.
10. Snarski E, Godal R, Siehl JM *et al.* Overexpression of paired box genes (PAX) 2 and 8 as potential basis for upregulation of Wilms' tumor gene 1 (WT1) in human carcinomas. Manuscript in preparation.
11. Boon T and Old LJ. Cancer Tumor antigens. Curr Opin Immunol 9:681-683; 1997.
12. Scheibenbogen C, Sun Y, Keilholz U et al. Identification of known and novel immunogenic T-cell epitopes from tumor antigens recognized by peripheral blood T cells from patients responding to IL-2-based treatment. Int J Cancer 98:409-414; 2002.
13. Hebart H, Daginik S, Stevanovic S et al. Sensitive detection of human cytomegalovirus peptide-specific cytotoxic T-lymphocyte responses by interferon-gamma-enzyme-linked immunospot assay and flow cytometry in healthy individuals and in patients after allogeneic stem cell transplantation. Blood 99:3830-3837; 2002
14. Reynolds SR, Celis E, Sette A et al. Identification of HLA-A*03, A*11 and B*07-restricted melanoma-associated peptides that are immunogenic in vivo by vaccine-induced immune response (VIIR) analysis. J Immunol Methods 244:59-67; 2000.
15. Provenzano M, Mocellin S, Bettinotti M et al.. Identification of immune dominant cytomegalovirus epitopes using quantitative real-time polymerase chain reactions to measure interferon-gamma production by peptide-stimulated peripheral blood mononuclear cells. J Immunother 25:342-351; 2002.
16. Rammensee H, Bachmann J, Emmerich NP et al. SYFPEITHI: database for MHC ligands and peptide motifs. Immunogenetics 50:213-219; 1990.
17. Parker KC, Bednarek MA and Coligan JE. Scheme for ranking potential HLA-A2 binding peptides based on independent binding of individual peptide side-chains. J Immunol 152:163; 1994.
18. Kessler JH, Beekman NJ, Bres-Vloemans SA et al.. Efficient identification of novel HLA-A(*)0201-presented cytotoxic T lymphocyte epitopes in the widely expressed tumor antigen PRAME by proteasome-mediated digestion analysis. J Exp Med 193:73-88; 2001.
19. Lucchiari-Hartz M, van Endert PM, Lauvau G et al. Cytotoxic T lymphocyte epitopes o HIV-1 Nef: Generation of multiple definitive major histocompatibility complex class I ligands by proteasomes. JExp Med 191:239-252; 2000.
20. Scheibenbogen C, Sun Y, Keilholz U et al. Identification of known and novel immunogenic T-cell epitopes from tumor antigens recognized by peripheral blood T cells from patients responding to IL-2-based treatment. Int J Cancer 98:409-414; 2002.
21. Hebart H, Daginik S, Stevanovic S et al. Sensitive detection of human cytomegalovirus peptide-specific cytotoxic T-lymphocyte responses by interferon-gamma-enzyme-linked immunospot assay and flow cytometry in healthy individuals and in patients af ter allogeneic stem cell transplantation. Blood 99:3830-3837; 2002.
22. Reynolds SR, Celis E, Sette A et al. Identification of HLA-A*03, A*11 and B*07-restricted melanoma-associated peptides that are immunogenic in vivo by vaccine-induced immune response (VIIR) analysis. J Immunol Methods 244:59-67; 2000.
23. Scheibenbogen C, Schadendorf D, Bechrakis N et al. Effects of granulocyte-macrophage colony stimulating factor and foreign helper protein as immunologic adjuvans on the T-cell response to vaccination with tyrosinase peptides. Int J Cancer 104:188-194; 2003.

## Claims

1. An immunogenic T-cell epitope represented by a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

2. A use of an immunogenic T-cell epitope from PAX2 and/or PAX8, preferably from PAX2, as a target for recognition by a targeting means.

3. The use according to claim 2, wherein the T-cell epitope is a HLA binding T-cell epitope, preferably selected from a HLA-A2, HLA-A or HLA-A24 binding T-cell epitope.

4. The use according to claim 2 or 3, wherein the T-cell epitope is represented by a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

5. The use according to claim 4, wherein the T-cell epitope is represented by the peptide according to SEQ ID No. 5 (3496).

6. The use according to claim 4, wherein three T-cell epitopes are used represented by the peptides according to SEQ ID No. 16 (3520), SEQ ID. No 29 (3550), and SEQ ID No. 32 (3553).

7. The use according to claim 4, wherein two T-cell epitopes are used represented by the peptides according to SEQ ID No. 5 (3496) and SEQ ID No. 6 (3497).

8. The use according to claim 4, wherein two T-cell epitopes are used represented by the peptides according to SEQ ID No. 20 (3519) and SEQ ID No. 21 (3518).

9. The use according to any of claims 2 to 8, wherein the targeting means is a component of the immune system, preferably a T-cell or an antibody.

10. A use of a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553) for the preparation of a pharmaceutical composition for the treatment of a PAX2 and/or PAX8 expressing disease, preferably a PAX2 expressing disease.

11. The use according to claim 10, wherein the disease is selected from renal cancer, colorectal cancer, breast cancer, and ovarian cancer.

12. The use according to claim 10, wherein the peptide according to SEQ ID No. 5 (3496) is used for the preparation, and the disease preferably is renal cancer.

13. The use according to claim 10, wherein the peptides according to SEQ ID No. 16 (3520), SEQ ID No. 29 (3550), and SEQ ID No. 32 (3553) are used for the preparation, and the disease preferably is colorectal cancer.

14. The use according to claim 10, wherein the peptides according to SEQ ID No. 5 (3496) and 3497 SEQ ID No. 6 (3497) are used for the preparation, and the disease preferably is colorectal cancer.

15. The use according to claim 10, wherein the peptides according to SEQ ID No. 20 (3519) and SEQ ID No. 21 (3518) are used for the preparation, and the disease pref erably is colorectal cancer.

16. A use of a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553) for the preparation of a pharmaceutical composition for raising an immune,response in a subject, preferably a mammal.

17. The use according to claim 16, wherein the immune response is raised by contacting the pharmaceutical composition with T-cells of a subject, preferably a mammal, *in vivo.*

18. The use according to claim 16, wherein the immune response is raised by contacting the pharmaceutical composition with T-cells of a subject, preferably a mammal, *ex vivo.*

19. A pharmaceutical composition comprising a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

20. A use of a targeting means for the preparation of a pharmaceutical composition for the treatment of a PAX2 and/or PAX8-expressing disease, preferably a PAX2-expressing disease, most preferably selected from renal cancer, colorectal cancer, breast cancer, and ovarian cancer.

21. The use according to claim 20, wherein the targeting means is a T-cell or an antibody directed against an immunogenic T-cell epitope from PAX2 and/or PAX8, preferably from PAX2.

22. The use according to claim 21, wherein the T-cell epitope is represented by a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

23. A pharmaceutical composition comprising a targeting means, preferably a T-cell or an antibody directed against a an immunogenic T-cell epitope from PAX2 and/or PAX8 preferably from PAX2.

24. The use according to claim 23, wherein the T-cell epitope is represented by a peptide selected from the group consisting of peptides according to SEQ ID No. 5 (3496), SEQ ID No. 6 (3497), SEQ ID No. 16 (3520), SEQ ID No. 20 (3519), SEQ ID No. 21 (3518), SEQ ID No. 29 (3550), and SEQ ID No. 32 (3553).

25. A method for identifying an immunogenic T-cell epitope, a portion or a variant thereof comprising the following steps:
(a) predicting a candidate epitope from PAX2 and/or PAX8, preferably from PAX2;
(b) *ex vivo* screening of one or more candidate epitopes predicted in step (a) for recognition by T-cells; and
(c) *in vitro* generating of T-cell clones.

26. The method according to claim 25, wherein the T-cell epitope is a HLA binding T-cell epitope, preferably selected from HLA-A2, HLA-A and HLA-A24 binding T-cell epitope.

27. The method according to claim 25 or 26, wherein the candidate epitope is excluded from the highly conserved paired box sequence of PAX2 and/or PAX8, preferably from PAX2.

28. The method according to any of claims 25 to 27, wherein the T-cells in step (b) are obtained from a tumour patient having a PAX2 and/or PAX8-expressing disease, preferably a PAX2-expressing disease, most preferably selected from renal cancer, colorectal cancer, breast cancer and ovarian cancer.

29. The method according to any of claims 25 to 28, wherein the T-cell clones are useful for demonstrating natural epitope processing.


SEQUENCE LISTING

<110> Charité - Universitätsmedizin Berlin

<120> PAX2 and PAX8 as Tumour Targets for Immunologic and Molecular
      Treatment Strategies

<130> FB16819

<160> 37

<170> PatentIn version 3.3

<210> 1
<211> 9
<212> PRT
<213> human

<400> 1

Ala Leu Thr Pro Gly Leu Asp Glu Val
1               5


<210> 2
<211> 9
<212> PRT
<213> human

<400> 2

Ser Leu Pro Ala Leu Thr Pro Gly Leu
1               5


<210> 3
<211> 9
<212> PRT
<213> human

<400> 3

Gly Leu Asp Glu Val Lys Ser Ser Leu
1               5


<210> 4
<211> 9
<212> PRT
<213> human

<400> 4

His Leu Val Trp Thr Leu Arg Asp Val
1               5


<210> 5
<211> 9
<212> PRT
<213> human

<400> 5

Thr Leu Pro Gly Tyr Pro Pro His Val
1               5

<210>  6
<211>  9
<212>  PRT
<213>  human

<400>  6

Val Thr Ala Pro Gly His Thr Ile Val
1               5

<210>  7
<211>  9
<212>  PRT
<213>  human

<400>  7

Tyr Ser Ile Asn Gly Ile Leu Gly Ile
1               5

<210>  8
<211>  9
<212>  PRT
<213>  human

<400>  8

Gly Gly Leu His Leu Val Trp Thr Leu
1               5

<210>  9
<211>  9
<212>  PRT
<213>  human

<400>  9

Thr Leu Ala Gly Met Val Pro Gly Ser
1               5

<210>  10
<211>  9
<212>  PRT
<213>  human

<400>  10

Arg Gly Gly Gly Gly Leu His Leu Val
1               5

<210>  11
<211>  9
<212>  PRT

<213>  human

<400>  11

Thr Ala Ser Pro Pro Val Ser Ser Ala
1               5


<210>  12
<211>  9
<212>  PRT
<213>  human

<400>  12

Ser Ile Asn Gly Ile Leu Gly Ile Pro
1               5


<210>  13
<211>  9
<212>  PRT
<213>  human

<400>  13

Ala His Ile Arg Gly Gly Gly Gly Leu
1               5


<210>  14
<211>  9
<212>  PRT
<213>  human

<400>  14

Ile Arg Gly Gly Gly Gly Leu His Leu
1               5


<210>  15
<211>  9
<212>  PRT
<213>  human

<400>  15

Leu Ser Ala Ser Thr Asn Pro Glu Leu
1               5


<210>  16
<211>  10
<212>  PRT
<213>  human

<400>  16

Gly Val Thr Ala Pro Gly His Thr Ile Val
1               5                   10

<210>  17
<211>  9
<212>  PRT
<213>  human

<400>  17

Thr Ile Val Pro Ser Thr Ala Ser Pro
1               5


<210>  18
<211>  9
<212>  PRT
<213>  human

<400>  18

Glu Leu Gly Ser Asn Val Ser Gly Thr
1               5


<210>  19
<211>  9
<212>  PRT
<213>  human

<400>  19

Ser Ala Ala Pro Arg Ser Ala Pro Ala
1               5


<210>  20
<211>  10
<212>  PRT
<213>  human

<400>  20

Ser Leu Ser Ala Ser Thr Asn Pro Glu Leu
1               5                   10


<210>  21
<211>  10
<212>  PRT
<213>  human

<400>  21

Thr Leu Arg Asp Val Ser Glu Gly Ser Val
1               5                   10


<210>  22
<211>  9
<212>  PRT
<213>  human

<400>  22

His Ile Arg Gly Gly Gly Leu His Leu

1                   5


<210>  23
<211>  9
<212>  PRT
<213>  human

<400>  23

Ile Arg Gly Gly Gly Leu His Leu Val
1               5


<210>  24
<211>  10
<212>  PRT
<213>  human

<400>  24

Gln Gly Asn Glu Tyr Ser Leu Pro Ala Leu
1               5                   10


<210>  25
<211>  10
<212>  PRT
<213>  human

<400>  25

Pro Ala Leu Thr Pro Gly Leu Asp Glu Val
1               5                   10


<210>  26
<211>  10
<212>  PRT
<213>  human

<400>  26

Ser Thr Asn Pro Glu Leu Gly Ser Asn Val
1               5                   10


<210>  27
<211>  10
<212>  PRT
<213>  human

<400>  27

Thr Thr Leu Pro Gly Tyr Pro Pro His Val
1               5                   10


<210>  28
<211>  10
<212>  PRT
<213>  human

<400>  28

Leu Leu Ser Ser Pro Tyr Tyr Tyr Ser Ala
1               5                   10


<210>  29
<211>  10
<212>  PRT
<213>  human

<400>  29

Ser Thr Ala Ser Pro Pro Val Ser Ser Ala
1               5                   10


<210>  30
<211>  10
<212>  PRT
<213>  human

<400>  30

Leu His Leu Val Trp Thr Leu Arg Asp Ala
1               5                   10


<210>  31
<211>  10
<212>  PRT
<213>  human

<400>  31

Tyr Ser Leu Pro Ala Leu Thr Pro Gly Leu
1               5                   10


<210>  32
<211>  10
<212>  PRT
<213>  human

<400>  32

Ile Val Pro Ser Thr Ala Ser Pro Pro Val
1               5                   10


<210>  33
<211>  10
<212>  PRT
<213>  human

<400>  33

Gly Gly Gly Leu His Leu Val Trp Thr Leu
1               5                   10


<210>  34
<211>  10

<212>  PRT
<213>  human

<400>  34

Asn Val Ser Gly Thr Gln Thr Tyr Pro Val
1               5                   10


<210>  35
<211>  10
<212>  PRT
<213>  human

<400>  35

Ser Tyr Ser Ile Asn Gly Ile Leu Gly Ile
1               5                   10


<210>  36
<211>  10
<212>  PRT
<213>  human

<400>  36

Thr Leu Ala Gly Met Val Pro Gly Ser Glu
1               5                   10


<210>  37
<211>  10
<212>  PRT
<213>  human

<400>  37

Ser Ala Ala Pro Arg Ser Ala Pro Ala Ala
1               5                   10